# EUROPÄISCHE PATENTSCHRIFT

(11) **EP 1 962 733 B1**
(45) Veröffentlichungstag und Bekanntmachung des Hinweises auf die Patenterteilung: **17.08.2016**
(21) Anmeldenummer: 06828625.1
(22) Anmeldetag: 07.12.2006
(51) Int. Cl.: A61F 2/58

(54) **HANDPROTHESE SOWIE KRAFTÜBERTRAGUNGSEINRICHTUNG**
HAND PROSTHESIS AND FORCE TRANSMISSION DEVICE
PROTHESE DE MAIN ET DISPOSITIF DE TRANSFERT DE FORCE

(30) Priorität: 20.12.2005 DE 102005061266
(43) Veröffentlichungstag der Anmeldung: 03.09.2008
(73) Patentinhaber: Otto Bock HealthCare GmbH, 37115 Duderstadt (DE)
(72) Erfinder: PUCHHAMMER, Gregor, A-1130 Wien (AT)
(74) Vertreter: Stornebel, Kai
(86) Internationale Anmeldenummer: PCT/DE2006/002175
(87) Internationale Veröffentlichungsnummer: WO 2007/076763

(56) Entgegenhaltungen:
- EP-A1- 0 045 818
- DE-A1- 19 906 294
- FR-A1- 2 236 478
- GB-A- 1 571 140
- US-A- 4 685 924
- US-A- 4 685 929
- US-A- 4 921 293
- US-A- 4 955 918

## Beschreibung

Die Erfindung betrifft eine Handprothese mit einem Chassis, an dem zumindest zwei Fingerprothesen gelenkig gelagert sind, die über einen Antrieb, der mit den Fingerprothesen über eine Kraftübertragungseinrichtung verbunden ist, um zumindest eine Schwenkachse bewegbar sind sowie eine Kraftübertragungseinrichtung als solche.

Wenn eine Hand amputiert werden musste oder durch einen Unfall irreversibel von einem Arm abgetrennt wurde, kann die Optik und teilweise die Funktion der Hand durch eine Handprothese ersetzt werden. Dazu muss die Handprothese in der Lage sein, Greifeinrichtungen, die als Fingernachbildungen ausgebildet sein können, zueinander zu verlagern, um ein Greifen eines Gegenstandes zu ermöglichen.

Neben einem Zweifingergreifer, wie er aus der US 2004/0015240 A1 bekannt ist, werden Handprothesen vorgeschlagen, die über einen Antrieb verfügen, der über ein Kegelradgetriebe starr mit einem Handchassis verbunden ist. Je nach Drehrichtung des Antriebes werden dabei die Fingerprothesen aufeinander zu oder voneinander weg bewegt. Dieser Antrieb kann über myoelektrische Signale angesteuert werden. Eine solche Handprothese ist in der US 2005/0021154 A1 beschrieben. Die Anmutung einer solchen Handprothese ist wenig natürlich.

Die US 4,685,929 beschreibt eine Handprothese gemäß des Oberbegriffs des Anspruch 1.

Die DE 320 758 B1 beschreibt eine Kunsthand, deren Finger durch Gliederstangen, die sowohl als Zug- als auch als Druckorgane wirken, beug- und streckbar sind. Die Gelenkstange ist unterhalb der Gelenke der Fingerglieder angeordnet und besitzt an jedem ihrer beiden letzten Glieder je ein nach rückwärts über ihre Verbindungsscharniere hinausgehendes Verlängerungsstück, mit denen sie lose zwischen Fingeranschlägen auf der Stangenunterseite und den Gelenkzapfen auf der Stangenoberseite geführt wird. Bei einem Vorschieben der Gelenkstange erfolgt die Fingerstreckung, bei einer Rückwärtsbewegung eine Fingerbeugung nach Maßgabe des Profils des zu ergreifenden Gegenstandes.

Die EP 45 818 A1 beschreibt eine Handprothese mit einem Chassis, gelenkig daran gelagerter Finger sowie einer Kraftübertragungseinrichtung in Gestalt eines Motors und eines Schneckengetriebes, über das Wickeltrommeln angetrieben werden.

Die DE 199 06 249 A1 beschreibt eine Fingerprothese mit einem Chassis und einem Servomotor, der über Bowdenzüge die Fingerglieder antreibt.

Die US 908,881 A betrifft eine Kunsthand an einem Prothesenunterarm. An der Kunsthand sind über Gestänge, Ketten und Federn bewegbare Prothesenfinger angeordnet. Die Betätigung einzelner Prothesenfinger erfolgt über Ketten, die an einem Querträger befestigt sind. Über einen Gleitbolzen, ein Joch und ein Verbindungsteil wird ein Zugstab und eine Feder vorgespannt, um die Ketten zu bewegen und die Prothesenfinger zu aktuieren.

Aufgabe der vorliegenden Erfindung ist es, eine Handprothese und eine Kraftübertragungseinrichtung bereitzustellen, mit der eine dem natürlichen Erscheinungsbild einer Hand angenäherte Gestaltung und Funktion bereitgestellt werden kann.

Erfindungsgemäß wird diese Aufgabe durch eine Handprothese mit den Merkmalen des Anspruchs 1 sowie eine Kraftübertragungseinrichtung mit den Merkmalen des Anspruchs 12 gelöst. Vorteilhafte Ausgestaltungen und Weiterbildungen der Erfindung sind in den Unteransprüchen beschrieben.

Die erfindungsgemäße Handprothese mit einem Chassis, an dem zumindest zwei Fingerprothesen gelenkig gelagert sind, die über einen Antrieb, der mit den Fingerprothesen über eine Kraftübertragungseinrichtung verbunden ist, um zumindest eine Schwenkachse bewegbar sind, wobei die Kraftübertragungseinrichtung zwischen dem Antrieb und den Fingerprothesen drucknachgiebig oder biegeelastisch und zugstarr ausgebildet ist, sieht vor, dass mehrere Kraftübertragungseinrichtungen an einer gemeinsamen Lagerstelle gelagert sind und die Lagerstelle zu den Schwenkachsen der Fingerprothesen verlagerbar ist, dass die Kraftübertragungseinrichtung eine Seil- oder Faserkomponente oder Litze aufweist, wobei zwei Kraftübertragungseinrichtungen über eine gemeinsame Seil-, Faser, oder Litzenkomponente miteinander verbunden sind und dass die gemeinsame Seil-, Faser- oder Litzenkomponente an der gemeinsamen Lagestelle und an verschiedenen Fingerprothesen gelagert ist.

Während die herkömmlichen Handprothesen eine starre Kopplung zwischen Antrieb und Fingerprothesen vorsehen, ist durch die drucknachgiebige oder biegeelastische Kopplung des Antriebes mit den Fingerprothesen gewährleistet, dass auf Druckbelastung der Kraftübertragungseinrichtung, also bei einer Krafteinwirkung, die ein Schließen der Hand bzw. einer Verkleinerung des Winkels zwischen den Fingerprothesen und dem Handchassis bewirkt, die Fingerprothesen nachgeben und neben einer natürlicheren Anmutung auch eine Schonung der mechanischen Komponenten bewirkt. Die Schonung der Komponenten ist dadurch begründet, dass die mitunter erheblichen Kräfte, die bei einem zufälligen Anstoßen der Fingerprothesen an Gegenstände entstehen, nicht unmittelbar über die Kraftübertragungseinrichtung auf den Antrieb übertragen werden. Vielmehr werden aufgrund der biegeelastischen, vorzugsweise rückfedernden Ausgestaltung der Kraftübertragungseinrichtung eine Verlagerung und eine Umwandlung der auf die Fingerprothesen einwirkenden Kräfte in eine Bewegung ermöglicht. Diese Bewegung kann bis zum maximalen Beugewinkel der Fingerprothesen erfolgen.

Um dagegen eine ungehinderte und zuverlässige sowie präzise Schließbewegung der Fingerprothesen, ausgehend von einer geöffneten Grundstellung, sicherzustellen, ist die Kraftübertragungseinrichtung zugstarr ausgebildet, das heißt, dass Zugkräfte möglichst ohne Dehnung der Kraftübertragungseinrichtung übertragen werden. Dazu ist es vorgesehen, dass die Kraftübertragungseinrichtung eine Seil-, Litzen- oder Faserkomponente aufweist, über die die Zugkräfte übertragen werden. Diese Seil-, Litzen- oder Faserkomponente kann aus einem Drahtseil oder hochfesten Fasern wie Kohlefasern, Aramid oder Glasfasern hergestellt sein. Daneben können andere Kunststoff- oder Naturfasern Einsatz finden.

Die Erfindung sieht vor, dass mehrere Kraftübertragungseinrichtungen an einer gemeinsamen Lagerstelle gelagert sind und die Lagerstelle zu den Schwenkachsen der Fingerprothesen verlagerbar ist, um eine Beugen der Fingerprothesen zu bewirken. Die gemeinsame Lagerstelle kann unmittelbar an dem Antrieb oder an einem Abtriebselement eines Getriebes angeordnet sein. Durch die gemeinsame Lagerung kann eine Synchronität der Beugebewegungen erleichtert werden.

Zwei Kraftübertragungseinrichtungen sind über eine gemeinsame Seil-, Faser, oder Litzenkomponente miteinander verbunden, wobei die gemeinsame Seil-, Faser- oder Litzenkomponente an der gemeinsamen Lagestelle und an verschiedenen Fingerprothesen gelagert ist. Die Seil-, Faser- oder Litzenkomponente überträgt dann die Zugkraft, die von dem Antrieb über die gemeinsame Lagerstelle aufgrund der Verlagerung relativ zu den Schwenkachsen der Fingerprothesen erzeugt wird, auf die Fingerprothesen und bewirkt eine Beugung der Fingerprothesen. Dabei kann die Seil-, Faser- oder Litzenkomponente auf der Lagerstelle entlang gleiten und so eine gleichmäßige Beugung der beiden miteinander verbundenen Fingerprothesen sicherstellen.

Damit hohe Zugkräfte von dem Antrieb auf die Fingerprothesen übertragen werden können, ist die Seil- Litzen- oder Faserkomponente als eine geschlossene Schlaufe ausgebildet. Alternativ ist die Ausbildung einer offenen Schlaufe oder einer verdrehten Schlaufe vorgesehen. Die Verbindung der beiden offenen Seil- oder Litzenenden oder die Befestigung im übrigen Material oder an dem Antrieb oder der Fingerprothesen kann durch Verdrehung, Verspleißung oder Verklebung erfolgen. Wird nachfolgend von einer Seilkomponente gesprochen, sind Faser- oder Litzenkomponenten mit eingeschlossen.

Eine Weiterbildung der Erfindung sieht vor, dass die Kraftübertragungseinrichtung eine Elastomerkomponente aufweist, durch die es möglich ist, die Biegeelastizität der Kraftübertragungseinrichtung über einen weiten Bereich einzustellen. Durch die geeignete Wahl eines Elastomerwerkstoffes, der bevorzugt die Seilkomponente zumindest teilweise umschließt oder vollständig aufnimmt, ist es möglich, einen in Ruhelage formstabilen Körper herzustellen, der einerseits aufgrund der eingelagerten Seilkomponente sehr hohe Zugkräfte und andererseits aufgrund der Elastomerkomponente ein gewünschtes biegeelastisches und druckelastisches Verhalten aufweist.

Alternativ kann die Kraftübertragungseinrichtung als eine Feder-Dämpfer-Einheit ausgestaltet sein, insbesondere als eine Pneumatikeinheit, bei der auf Druckbelastung ein Luftvolumen komprimiert wird, das sich nach Wegfall der Druckkraft entspannt und eine Rückverlagerung eines Pneumatikkolbens und damit der Fingerprothesen bewirkt. Alternativ kann die Kraftübertragungseinrichtung als eine Feder ausgebildet sein, die ggf. vorgespannt ist.

Zur Ankopplung der Kraftübertragungseinrichtung an den Antrieb und die Fingerprothesen sind Lagerbuchsen vorgesehen, die in der Kraftübertragungseinrichtung eingebettet sind. Bevorzugt werden diese Lagerbuchsen von dem Elastomerelement bzw. der Elastomerkomponente umschlossen und befinden sich innerhalb der Seil- oder Faserkomponente, bevorzugt innerhalb der Seil- oder Faserschlaufe.

Um eine Rückstellbewegung von in Richtung Handchassisinnenfläche gebogenen Fingerprothesen realisieren zu können, ist die Kraftübertragungseinrichtung federelastisch ausgebildet, so dass die Fingerprothesen ohne Beaufschlagung mit einer Zugkraft durch den Antrieb oder durch ein mit dem Antrieb gekoppeltes Übertragungselement in eine Ausgangsstellung zurückbewegt wird. Diese Ausgangsstellung entspricht bevorzugt einer leicht geöffneten Hand. Die Kraftübertragungseinrichtung ist somit in der Lage, eine begrenzte Druckkraft zu übertragen. Durch eine entsprechende Anlenkung oder Ausbildung der Kraftübertragungseinrichtung kann eine leichte Überstreckung der Fingerprothesen, ausgehend von der Grundstellung, ermöglicht werden. Die Federrate der Kraftübertragungseinrichtung ist dabei so bemessen, dass bei einer Beaufschlagung der Kraftübertragungseinrichtung mit einer Druckkraft, also bei einem Verschwenken der Fingerprothesen in Richtung Handchassisinnenfläche, eine Rückstellung der Fingerprothesen in die Ausgangsstellung bewirkt wird. Die Rückstellkraft muss daher so groß sein, dass die Halte- und Reibungskräfte innerhalb der Handprothese überwunden werden.

Die Kraftübertragungseinrichtungen können an einer Wippe befestigt sein, die an der gemeinsamen Lagerstelle gelagert ist. Die Kraftübertragungseinrichtungen können in unterschiedlichen Abständen zu der gemeinsamen Lagerstelle an der Wippe befestigt sein, um eine angepasste Verlagerung der jeweiligen Fingerprothesen an die geometrischen Bedingungen innerhalb der Handprothese oder an die Einsatzbedingungen ermöglichen zu können.

Die Kraftübertragungseinrichtungen können in mehreren Orientierungen biegeelastisch ausgebildet, so dass bei einer Druckbelastung in Richtung der Längsachse die Lagerstellen zueinander verlagerbar sind. Die allseitig biegbare Kraftübertragungseinrichtung wirkt dann wie ein Kugelgelenk und gleicht Abweichungen von der idealen Achsstellung aus. Gegebenenfalls kann auf eine drehbare Lagerung um Achsen verzichtet werden.

Die Kraftübertragungseinrichtung zur Übertragung von Kräften von einem Antrieb einer Handprothese auf gelenkig an einem Handchassis gelagerten Fingerprothesen ist wie oben beschrieben ausgebildet und ermöglicht eine leichte und preiswerte Kopplung von Antrieb und Fingerprothesen sowie eine wirksame Übertragung von Zugkräften. Darüber hinaus wird eine entgegen einer ungewollten Belastung nachgiebige Lagerung und eine einfache Rückstellung mit der Kraftübertragungseinrichtung erreicht.

Nachfolgend wird ein Ausführungsbeispiel der Erfindung anhand der beigefügten Figuren näher erläutert. Gleiche Bezugszeichen in den unterschiedlichen Figuren bezeichnen gleiche Elemente. Es zeigen:
- Figur 1-: eine schematische Darstellung einer Handprothese;
- Figur 2-: eine schematische Teildarstellung des Funktionsaufbaus einer Handprothese;
- Figuren 3a - 3d -: eine Kraftübertragungseinrichtung in unterschiedlichen Ansichten;
- Figur 4 -: eine Detaildarstellung einer Wippe;
- Figur 5 -: eine Detaildarstellung aus Figur 2; sowie
- Figur 6 -: die erfindungsgemäße Ausführungsvariante der Figur 5.

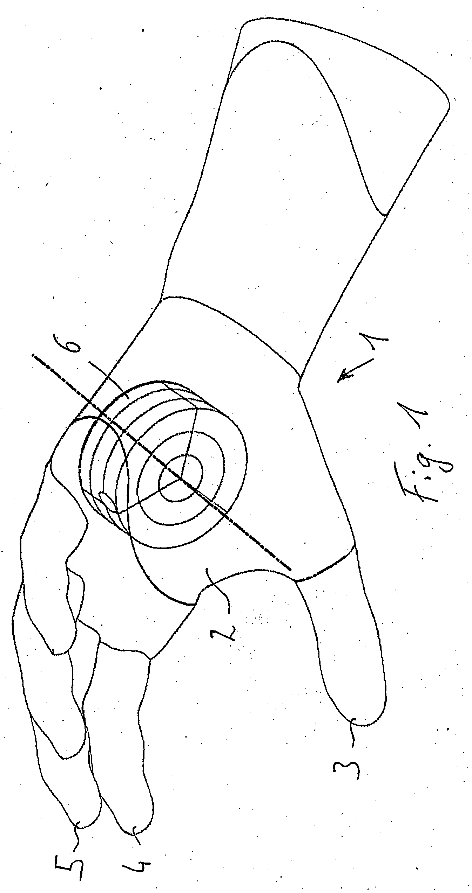

In der Figur 1 ist eine Handprothese 1, bestehend aus einem Handchassis 2 und zumindest drei gelenkig an dem Handchassis 2 gelagerten Fingerprothesen 3, 4, 5 gezeigt. Die Fingerprothesen 3, 4, 5 entsprechen dem Daumen, Zeigefinger und Mittelfinger einer natürlichen Hand. Eine bewegliche und über einen Antrieb 6 aktuierbare Lagerung dieser drei Fingerprothesen 3, 4, 5 reicht aus, um die Mehrzahl der Greifverrichtungen einer Hand ausführen zu können. Die beiden übrigen Finger, der Ringfinger und der kleine Finger, können passiv mitbewegt werden und aus einem Elastomermaterial bestehen, um eine möglichst natürliche Anmutung zu erzielen. Innerhalb des Handchassis 2 ist der Antrieb 6 in Gestalt eines Elektromotors mit einem dazugehörigen Getriebe gelagert. Ebenfalls kann eine nicht dargestellte Energiequelle für den Antrieb 6 innerhalb des Handchassis 2 angeordnet sein. Eine Ansteuerung des Antriebs 6 erfolgt über ein Steuergerät, das ebenfalls in dem Handchassis 2 angeordnet sein kann. Die entsprechenden Signale können über eine Fernbedienung generiert oder als myoelektrische Signale ausgebildet sein.

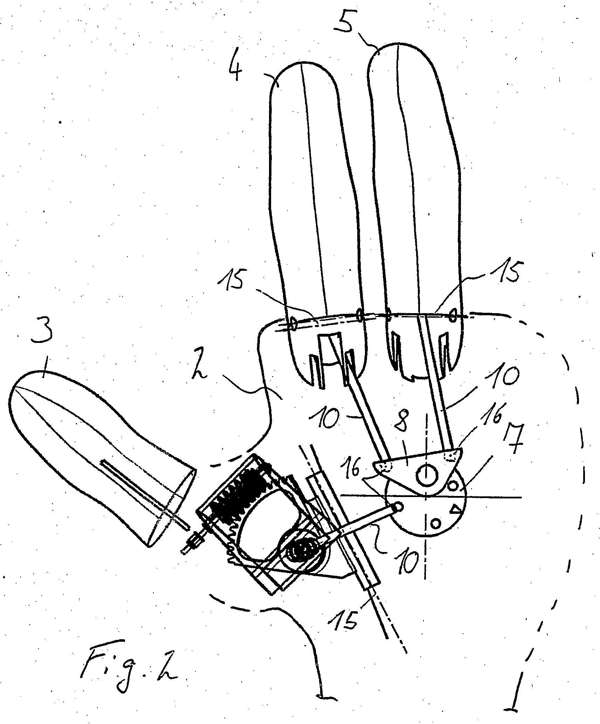

In der Figur 2 ist eine schematische Darstellung der Funktionsweise der Handprothese 1 gezeigt. An dem Handchassis 2 sind die drei Fingerprothesen 3, 4, 5 um Gelenkachsen 15 verschwenkbar gelagert. Die Fingerprothesen 3, 4, 5 sind über Kraftübertragungseinrichtungen 10, deren Aufbau weiter unten detailliert beschrieben werden wird, mit einer Drehscheibe 7 verbunden, die von dem Elektromotor 6 angetrieben wird. Die Kraftübertragungseinrichtungen 10 sind an der Drehscheibe 7 auf Achsen 16 entweder direkt oder über eine Wippe 8 gelagert. Der Zeigefinger 4 und der Ringfinger 5 sind über die Wippe 8, die drehbar auf der Drehscheibe 7 gelagert ist, miteinander gekoppelt. Die Drehscheibe 7 selbst ist entweder unmittelbar auf einer Abtriebswelle des Antriebes 6 oder einer Getriebeausgangswelle gelagert. Wird der Antrieb 6 aktiviert, wird die Drehscheibe 7 um einen entsprechenden Drehwinkel bewegt. Dadurch verlagern sich die Achsen 16 relativ zu den Schwenkachsen 15 der Fingerprothesen 3, was aufgrund der zugstarren Ausbildung der Kraftübertragungseinrichtungen 10 und einer von den Drehachsen 15 beabstandeten Anlenkung der Kraftübertragungseinrichtungen 10 an den Fingerprothesen 3, 4, 5 zu einer Verschwenkung der Fingerprothesen 3, 4, 5 führt. Wird der Antrieb 6 reversiert und bewegt sich die Drehscheibe 7 in eine Position, in der die Achsen 16 eine minimale Entfernung zu den Schwenkachsen 15 der Fingerprothesen 3, 4, 5 aufweisen, ist die geöffnete Ausgangsstellung erreicht. Durch die federelastischen Eigenschaften der Kraftübertragungseinrichtungen 10 werden dann die Fingerprothesen 3, 4, 5 in ihre geöffnete Ausgangsstellung bewegt. Dabei ist es vorgesehen, dass die Kraftübertragungseinrichtungen 10 wesentlich höhere Zugkräfte als Druckkräfte übertragen können. Dies entspricht den physiologischen Gegebenheiten einer natürlichen Hand, die beim Schließen der Hand wesentlich größere Kräfte als beim Öffnen aufbringen kann. Aus Gründen der Übersichtlichkeit sind der Ringfinger und der kleine Finger nicht dargestellt, diese können passiv an dem Mittelfinger 5 angelenkt und dadurch mitbewegt werden.

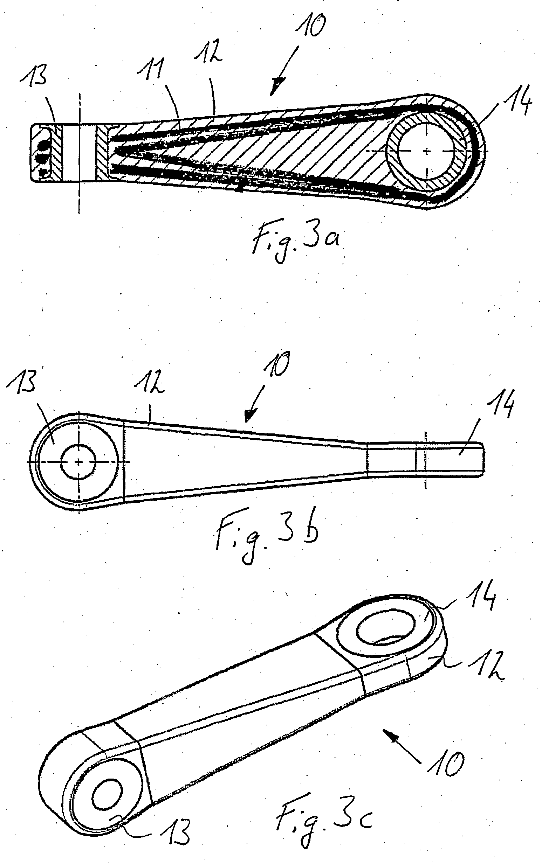

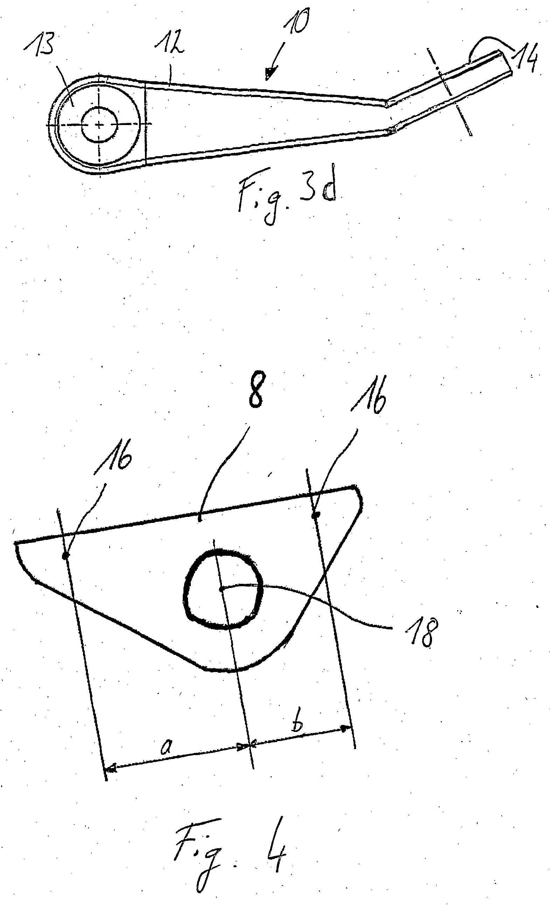

In der Figur 3 ist in einer Schnittdarstellung eine Kraftübertragungseinrichtung 10 gezeigt. Diese besteht aus einer Seil- oder Faserkomponente 11, die vorliegend als eine Schlaufe ausgebildet ist. Die Seilkomponente 11 kann aus mehreren Litzen oder Einzelschlaufen bestehen und als Stahlseil oder Kunststoffseil oder aus einem anderen, hochfesten Faserwerkstoff bestehen. Die Seilkomponente 11 ist in einem Elastomerelement 12 eingebettet, wodurch die Kraftübertragungseinrichtung 10 eine formstabile, jedoch biegeelastische Gestalt erhält. Die Elastomerkomponente 12 kann aus einem Silikon, einem Kautschuk oder einem anderen, elastischen Werkstoff bestehen. Trotz der Formstabilität ist durch die Biegsamkeit der Seil- oder Faserkomponente 11 und dem druckelastischen oder biegeelastischen Verhalten der Elastomerkomponente 12 eine Verformung, insbesondere Biegung aufgrund auf die Kraftübertragungseinrichtung 10 einwirkender Druckkräfte möglich. Dadurch können die über die Kraftübertragungseinrichtung 10 mit dem Antrieb 6 bzw. der Drehscheibe 7 gekoppelten Fingerprothesen 3, 4, 5 in Richtung auf die Handchassisinnenfläche verlagert werden, wobei eine Rückverlagerung aufgrund des federelastischen Verhaltens der Kraftübertragungseinrichtungen 10 erfolgt, wenn die entsprechende Gegenkraft entfällt.

Innerhalb der als Schlaufe ausgebildeten Seil- oder Faserkomponente 11 sind zwei Lagerbuchsen 13, 14 angeordnet, die ebenfalls in der Elastomerkomponente 12, beispielsweise einem Silikonwerkstoff, eingebettet sind. Die Lagerbuchsen 13, 14 werden auf entsprechenden Achsen an den Fingerprothesen 3, 4, 5 und auf Achsen 16 an der Drehscheibe 7 bzw. der Wippe 8 gelagert. Die Lagerbuchsen 13, 14 bestehen beispielsweise aus Bronze, um eine Gleitlagerung mit den entsprechenden Achsen 16 auszubilden. Aus Gründen der Übersichtlichkeit sind die Kopplungsachsen an den Fingerprothesen 3, 4, 5 nicht dargestellt. Diese Kopplungsachsen liegen beabstandet zu den Drehachsen 15, so dass durch Aufbringung von Zugkräften über die Kraftübertragungseinrichtungen 10 ein Moment um die Drehachsen 15 erzeugt wird, was zu einer korrespondierenden Verlagerung der Fingerprothesen 3, 4, 5 führt.

In den Figuren 3b und 3c ist zu erkennen, dass die Drehachsen der Lagerbuchsen 13, 14 senkrecht zueinander stehen, was in der konkreten Anordnung der Drehscheibe 7 und der darauf angeordneten oder ihr zugeordneten Achsen 16 begründet ist. Die Drehachsen der Lagerbuchsen 13, 14 können auch parallel oder in einem anderen Winkel zueinander ausgerichtet sein.

Ebenfalls ist in den Figuren 3a bis 3c zu erkennen, dass die Seil- oder Faserkomponente 11 vollständig in dem Elastomer 12 eingebettet sind, wodurch einerseits die Seil- oder Faserkomponente 11 vor äußeren Einflüssen geschützt und andererseits die Formstabilität der Kraftübertragungseinrichtung 10 erhöht wird. Die Figur 3d zeigt eine verformte Gestalt der Kraftübertragungseinrichtung 10, die in Längsrichtung auf Druck belastet wurde und ausknickte. Die Lagerbuchse 14 steht nicht mehr wie in den Figuren 3a bis 3c dargestellt in einem rechten Winkel zu der Lagerbuchse 13, vielmehr wird durch die Deformation eine windschiefe Stellung der Lagerbuchse 13, 14 zueinander und damit der Drehachsen 15,16 bewirkt. Die Kraftübertragungseinrichtung 10 wirkt aufgrund der elastischen Verformbarkeit in mehreren Orientierungen als ein Kugelgelenk und kann auch Achsenschiefstellungen, beispielsweise aufgrund von Fertigungstoleranzen ausgleichen.

Alternativ zu der dargestellten Ausführungsform kann die Kraftübertragungseinrichtung 10 auch aus einem anderen, drucknachgiebigen, z.B. federelastischen und zugstarren Element hergestellt sein, beispielsweise einem federnden Knick- oder Auslenkstab oder einer entsprechend konstruierten Drahtschlaufe.

Durch die druckelastische Lagerung findet keine unmittelbare Übertragung von Stoßkräften über die Fingerprothesen 3, 4. 5 auf den Antrieb 6 bzw. die Drehscheibe 7 statt. Vielmehr werden unbeabsichtigte Anstoßbewegungen abgefedert und gedämpft. Dies erhöht neben einer natürlichen Anmutung der Handprothese 1 auch die Lebensdauer der Lagerungen und Antriebskomponenten, beispielsweise bei einem Sturz.

Alternativ zu der dargestellten Ausführungsform kann die Kraftübertragungseinrichtung auch als eine Feder-Dämpfereinheit mit einer entsprechenden Steuerung ausgestattet sein, beispielsweise über einen pneumatischen oder hydraulischen Zylinder mit entsprechender Ventilsteuerung, die Zugkräfte wirksam übertragen können, bei Druckkräften jedoch eine elastische Nachgiebigkeit vorsehen. Durch eine pneumatische Ausgestaltung wird eine Rückverlagerung der nach innen gebogenen Fingerprothesen bewirkt.

Die Elastomerkomponente kann bei einer ausreichend biegeelastischen Ausgestaltung der Seilkomponente weggelassen werden; bei einer ausreichenden Zugfestigkeit der Elastomerkomponente kann diese als alleinige Kraftübertragungseinrichtung ausgebildet sein.

In der Figur 4 ist die Wippe 8 in vergrößerter Einzeldarstellung gezeigt. Die Wippe 8 weist eine gemeinsame Lagerstelle 18 in Gestalt eine Buchse auf, die auf eine korrespondierende Achse auf der nicht dargestellten Drehscheibe 7 aufgesteckt werden kann. Die Wippe 8 kann drehbar auf dieser Achse um die gemeinsame Lagerstelle 18 gelagert sein und weist an den Drehachsen 16 Befestigungsstellen für nicht dargestellte Kraftübertragungseinrichtungen 10 auf. Die Abstände a, b der Drehachsen 16 bzw. Befestigungsstellen von dem Drehzentrum der gemeinsamen Lagerstelle 18 können unterschiedlich ausgebildet sein, um den geometrischen oder mechanischen Gegebenheiten innerhalb der Handprothese Rechnung tragen zu können.

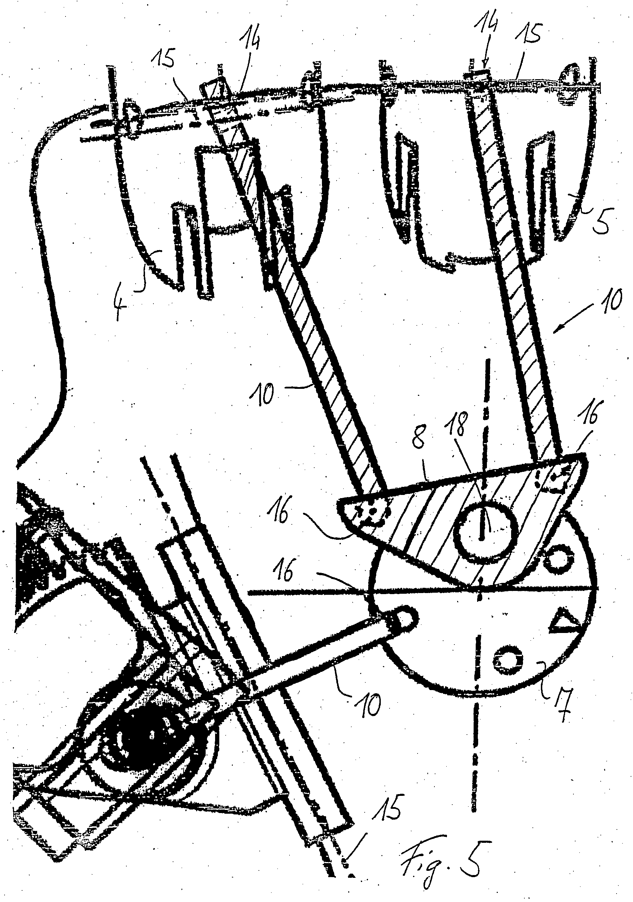

In der Figur 5 ist ein Ausschnitt der Figur 2 in einem vergrößerten Maßstab dargestellt. Gleiche Bezugszeichen bezeichnen gleiche Bauelemente. Der Figur 5 ist zu entnehmen, dass zwei Kraftübertragungseinrichtungen 10, die separat ausgeführt sind, über die Wippe 8 an der gemeinsamen Lagestelle 18 gelagert sind. Alternativ zu der Ausgestaltung gemäß Figur 5 können die Kraftübertragungseinrichtungen 10 auch ohne Wippe 8 an der Drehscheibe 7 an einem gemeinsamen Lagerungspunkt oder einer gemeinsamen Achse gelagert sein. Die den Fingerprothesen 4, 5 zugeordneten Lagerbuchsen 14 stehen in einem im Wesentlichen rechten Winkel zu den Drehachsen 16 an der Wippe 8, können jedoch, wie dem Zeigefinger 4 angedeutet, auch windschief dazu ausgerichtet sein.

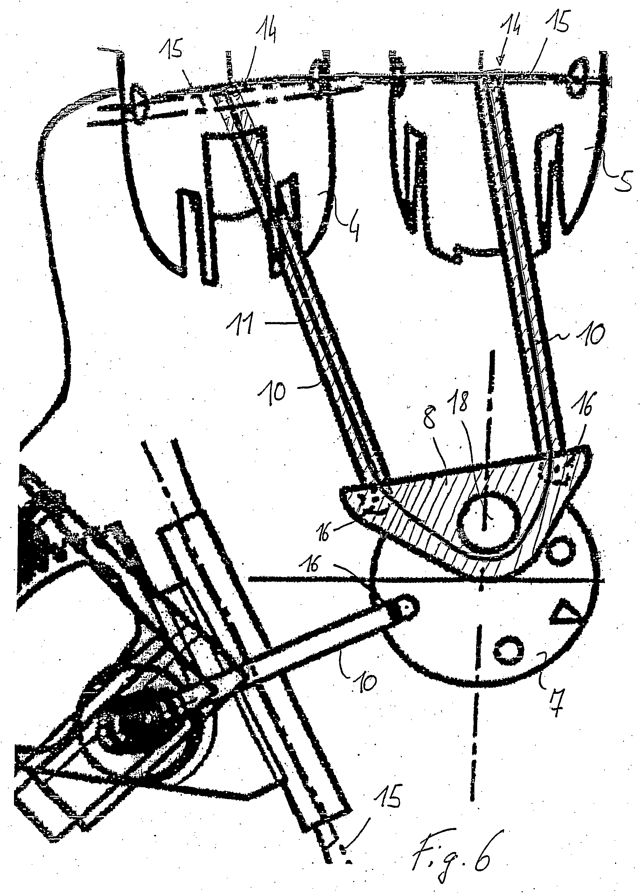

In der Figur 6 ist die erfindungsgemäße Variante der Ausgestaltung der Figur 5 gezeigt, bei der die Seilkomponente 11 als eine offene Schlaufe ausgebildet ist, die an den Lagerbuchsen 14 beispielsweise durch Verschweißen oder Ankleben befestigt ist. Die Seilkomponente 11 verbindet die beiden Lagerbuchsen 14 der Zeige- und Mittelfingerprothese 4, 5, wobei sie teilweise in die Elastomerkomponente der Kraftübertragungseinrichtungen 10 eingebettet ist. Im Bereich der Wippe 8 ist die Seilkomponente 11 hinter der gemeinsamen Lagerstelle 18 bzw. um die Drehachse auf der Drehscheibe 7 herumgeführt und nimmt die Zugkräfte aufgrund der Verlagerung der gemeinsamen Lagerstelle 18 relativ zu den Drehachsen 15 auf. Diese Zugkräfte werden durch die Seilkomponente 11 auf die Lagerbuchsen 14 übertragen und bewirken aufgrund der beabstandeten Lagerung der Lagerbuchsen 14 zu den Drehachsen 15 ein Moment um die Drehachsen 15, wodurch die Fingerprothesen 4, 5 gebeugt werden.

## Patentansprüche

1. Handprothese mit einem Chassis (2), an dem zumindest zwei Fingerprothesen (3, 4, 5) gelenkig gelagert sind, die über einen Antrieb (6), der mit der Fingerprothese (3, 4, 5) über eine Kraftübertragungseinrichtung (10) verbunden ist, um zumindest eine Schwenkachse bewegbar ist, wobei die Kraftübertragungseinrichtung (10) zwischen dem Antrieb (6) und der Fingerprothese (3, 4, 5) zugstarr und drucknachgiebig oder biegeelastisch ausgebildet ist, **dadurch gekennzeichnet, dass** mehrere Kraftübertragungseinrichtungen (10) an einer gemeinsamen Lagerstelle (18) gelagert sind und die Lagerstelle (18) zu den Schwenkachsen (15) der Fingerprothesen (3, 4, 5) verlagerbar ist, dass die Kraftübertragungseinrichtung (10) eine Seil- oder Faserkomponente (11) oder Litze aufweist, wobei zwei Kraftübertragungseinrichtungen (10) über eine gemeinsame Seil-, Faser-, oder Litzenkomponente (11) miteinander verbunden sind und dass die gemeinsame Seil-, Faser- oder Litzenkomponente (11) an der gemeinsamen Lagerstelle (18) und an verschiedenen Fingerprothesen (3, 4, 5) gelagert ist.

2. Handprothese nach Anspruch 1, **dadurch gekennzeichnet, dass** die Seil- oder Faserkomponente (11) oder Litze als geschlossene, offene oder verdrehte Schlaufe ausgebildet ist.

3. Handprothese nach einem der voranstehenden Ansprüche, **dadurch gekennzeichnet, dass** die Kraftübertragungseinrichtung (10) eine Elastomerkomponente (12) aufweist.

4. Handprothese nach Anspruch 3, **dadurch gekennzeichnet, dass** die Elastomerkomponente (12) die Seil- oder Faserkomponente (11) oder Litze zumindest teilweise umschließt.

5. Handprothese nach einem der voranstehenden Ansprüche, **dadurch gekennzeichnet, dass** die Kraftübertragungseinrichtung (10) federelastisch ausgebildet ist.

6. Handprothese nach Anspruch 5, **dadurch gekennzeichnet, dass** die Federrate der Kraftübertragungseinrichtung (10) so bemessen ist, dass bei einer Beaufschlagung der Kraftübertragungseinrichtung (10) mit einer Druckkraft eine Rückstellung der Fingerprothese (3, 4, 5) in eine Ausgangsstellung bewirkt wird.

7. Handprothese nach Anspruch 1, **dadurch gekennzeichnet, dass** die Kraftübertragungseinrichtung (10) als Feder-Dämpfer-Einheit, insbesondere Pneumatik- oder Hydraulikeinheit oder als eine Feder ausgebildet ist.

8. Handprothese nach einem der voranstehenden Ansprüche, **dadurch gekennzeichnet, dass** die Kraftübertragungseinrichtung (10) Lagerbuchsen (13, 14) zur Aufnahme von Achsen (15, 16) aufweist, die dem Handchassis (2) und der Fingerprothese (3, 4, 5) zugeordnet sind.

9. Handprothese nach einem der voranstehenden Ansprüche, **dadurch gekennzeichnet, dass** die Kraftübertragungseinrichtungen (10) an einer Wippe (8) befestigt sind, die an der gemeinsamen Lagerstelle (18) gelagert ist.

10. Handprothese nach Anspruch 9, **dadurch gekennzeichnet, dass** die Kraftübertragungseinrichtungen (10) in unterschiedlichen Abständen (a, b) zu der gemeinsamen Lagerstelle (18) an der Wippe (8) befestigt sind.

11. Handprothese nach einem der voranstehenden Ansprüche, **dadurch gekennzeichnet, dass** die Kraftübertragungseinrichtung (10) in mehreren Orientierungen biegeelastisch ausgebildet ist.

12. Kraftübertragungseinrichtung (10), für eine Handprothese (1) nach einem der voranstehenden Ansprüche, zur Übertragung von Kräften von einem Antrieb (6) der Handprothese (1) auf gelenkig an einem Handchassis (2) gelagerten Fingerprothesen (3, 4, 5).

## Claims

1. Hand prosthesis comprising a chassis (2), to which at least two finger prostheses (3, 4, 5) are articulated, said finger prosthesis being movable about at least one swiveling axis by means of a drive (6) which is connected to the finger prosthesis (3, 4, 5) by means of a force transmission unit (10), the force transmission unit (10) being formed between the drive (6) and the finger prosthesis (3, 4, 5) so as to be rigid under tension and yielding under pressure or elastic under bending, **characterized in that** a number of force transmission units (10) are mounted at a common bearing point (18) and the bearing point (18) is displaceable in relation to the swiveling axes (15) of the finger prostheses (3, 4, 5), **in that** the force transmission unit (10) has a cable or fiber component (11) or a stranded wire, two force transmission units (10) being connected to each other by means of a common cable, fiber or stranded-wire component (11) **in that** the common cable, fiber or stranded-wire component (11) is mounted at the common bearing point (18) and on different finger prostheses (3, 4, 5).

2. Hand prosthesis according to Claim 1, **characterized in that** the cable or fiber component (11) or the stranded wire is formed as a closed, open or twisted loop.

3. Hand prosthesis according to one of the preceding claims, **characterized in that** the force transmission unit (10) has an elastomer component (12).

4. Hand prosthesis according to Claim 3, **characterized in that** the elastomer component (12) at least partially encloses the cable or fiber component (11) or the stranded wire.

5. Hand prosthesis according to one of the preceding claims, **characterized in that** the force transmission unit (10) is formed in a resiliently elastic manner.

6. Hand prosthesis according to Claim 5, **characterized in that** the spring rate of the force transmission unit (10) is set such that, when the force transmission unit (10) is subjected to the force of a pressure, a return of the finger prosthesis (3, 4, 5) into the starting position is brought about.

7. Hand prosthesis according to Claim 1, **characterized in that** the force transmission unit (10) is formed as a spring-damper unit, in particular a pneumatic or hydraulic unit, or as a spring.

8. Hand prosthesis according to one of the preceding claims, **characterized in that** the force transmission unit (10) has bearing bushes (13, 14) for receiving spindles (15, 16), which are assigned to the hand chassis (2) and the finger prosthesis (3, 4, 5).

9. Hand prosthesis according to one of the preceding claims, **characterized in that** the force transmission units (10) are fastened to a rocker (8), which is mounted at the common bearing point (18).

10. Hand prosthesis according to Claim 9, **characterized in that** the force transmission units (10) are fastened to the rocker (8) at different distances (a, b) from the common bearing point (18).

11. Hand prosthesis according to one of the preceding claims, **characterized in that** the force transmission unit (10) is formed so as to be flexurally elastic in a number of orientations.

12. Force transmission unit (10) for a hand prosthesis according to one of the preceding claims, for the transmission of forces from a drive (6) of the hand prosthesis (1) to finger prostheses (3, 4, 5) articulated to a hand chassis (2).

## Revendications

1. Prothèse de main comprenant un châssis (2) sur lequel au moins deux prothèses de doigt (3, 4, 5) sont montées de manière articulée, qui sont mobiles autour d'au moins un axe de pivotement, au moyen d'un entraînement (6) qui est relié avec la prothèse de doigt (3, 4, 5) via un dispositif de transmission de force (10), dans laquelle le dispositif de transmission de force (10) entre l'entraînement (6) et la prothèse de doigt (3, 4, 5) est réalisé rigide vis-à-vis de la traction et flexible vis-à-vis de la pression ou élastique en flexion, **caractérisée en ce que** plusieurs dispositifs de transmission de force (10) sont montés sur un emplacement de montage commun (18), et l'emplacement de montage (18) est déplaçable vers les axes de pivotement (15) des prothèses de doigt (3, 4, 5), **en ce que** le dispositif de transmission de force (10) comprend un composant à câble ou à fibre (11) ou un toron, et deux dispositifs de transmission de force (10) sont reliés l'un à l'autre via un composant commun à câble, à fibre ou à toron (11), et **en ce que** le composant commun à câble, à fibre ou à toron (11) est monté sur l'emplacement de montage commun (18) et sur différentes prothèses de doigt (3, 4, 5).

2. Prothèse de main selon la revendication 1, **caractérisée en ce que** le composant à câble ou à fibre (11) ou le toron est réalisé sous forme d'une boucle fermée, ouverte ou torsadée.

3. Prothèse de main selon l'une des revendications précédentes, **caractérisée en ce que** le dispositif de transmission de force (10) comprend un composant en élastomère (12).

4. Prothèse de main selon la revendication 3, **caractérisée en ce que** le composant en élastomère (12) entoure au moins partiellement le composant à câble ou à fibre (11) ou le toron.

5. Prothèse de main selon l'une des revendications précédentes, **caractérisée en ce que** le dispositif de transmission de force (10) est réalisé avec l'élasticité d'un ressort.

6. Prothèse de main selon la revendication 5, **caractérisée en ce que** le taux d'élasticité du dispositif de transmission de force (10) est choisi de telle façon que lors de la sollicitation du dispositif de transmission de force (10) avec une force de pression, cela provoque un rappel de la prothèse de doigt (3, 4, 5) dans une position de départ.

7. Prothèse de main selon la revendication 1, **caractérisée en ce que** le dispositif de transmission de force (10) est réalisé sous forme d'unité à ressort-et-amortisseur, en particulier d'unité pneumatique ou hydraulique, ou sous forme d'un ressort.

8. Prothèse de main selon l'une des revendications précédentes, **caractérisée en ce que** le dispositif de transmission de force (10) comprend des douilles de montage (13, 14) pour la réception d'un axe (15, 16) qui sont associées au châssis (2) de la main et à la prothèse de doigt (3, 4, 5).

9. Prothèse de main selon l'une des revendications précédentes, **caractérisée en ce que** les dispositifs de transmission de force (10) sont fixés sur une bascule (8) qui est montée à l'emplacement de montage commun (18).

10. Prothèse de main selon la revendication 9, **caractérisée en ce que** les dispositifs de transmission de force (10) sont fixés sur la bascule (8) à des distances (a, b) différentes de l'emplacement de montage commun (18).

11. Prothèse de main selon l'une des revendications précédentes, **caractérisé en ce que** le dispositif de transmission de force (10) est réalisé élastique en flexion dans plusieurs orientations.

12. Dispositif de transmission de force (10), pour une prothèse de main (1) selon l'une des revendications précédentes, pour la transmission de forces depuis un entraînement (6) de la prothèse de main (1) vers des prothèses de doigt (3, 4, 5) montées de manière articulée sur un châssis (2) de la main.
